# FASCICULE DE BREVET EUROPEEN

(11) **EP 2 014 290 B1**
(45) Date de publication et mention de la délivrance du brevet: **23.01.2013**
(21) Numéro de dépôt: 08000893.1
(22) Date de dépôt: 21.10.2003
(51) Int. Cl.: A61K 31/444, A61P 1/04, A61P 11/00, A61P 11/04, A61P 11/06, A61P 11/14

(54) **Utilisation du ténatoprazole pour le traitment du reflux gastro-oesophagien**
Anwendung von Tenatoprazol zur Behandlung von Refluxösophagitis
Use of tenatoprazole for the treatment of gastroesophageal reflux disease

(30) Priorité: 21.10.2002 FR 0213113
(43) Date de publication de la demande: 14.01.2009
(62) Demande divisionnaire de: 03778427.9
(73) Titulaire: Sidem Pharma SA, 2613 Luxembourg (LU); Mitsubishi Tanabe Pharma Corporation, Osaka-shi (JP)
(72) Inventeur: Schutze, François, 78860 Saint-Nom-La-Bretèche (FR); Charbit, Suzy, 94000 Créteil (FR); Ficheux, Hervé, 94130 Nogent-sur-Marne (FR); Homerin, Michel, 91080 Courcouronnes (FR); Taccoen, Alain, 78150 Le Chesnay (FR); Inaba, Yoshio, Tokyo 103-8405 (JP)
(74) Mandataire: L'Helgoualch, Jean

(56) Documents cités:
- WO-A-00/50037
- WO-A-01/28558
- WO-A-98/16228
- WO-A-99/59544
- WO-A-02/072070
- ANON.: "Tenatoprazole: benatoprazole, TU 199" DRUGS IN R&D (2002), 3(4), 276-277, 2002, XP008018955
- UCHIYAMA KAZUYUKI ET AL: "The long-lasting effect of TU-199, a novel H+,K+-ATPase inhibitor, on gastric acid secretion in dogs." JOURNAL OF PHARMACY AND PHARMACOLOGY, vol. 51, no. 4, avril 1999 (1999-04), pages 457-464, XP008018962 ISSN: 0022-3573
- HOWDEN C W ET AL: "Appropriate acid suppression for optimal healing of duodenal ulcer and gastro-oesophageal reflux disease." SCANDINAVIAN JOURNAL OF GASTROENTEROLOGY, vol. 29, no. SUPPL. 201, 1994, pages 79-82, XP008018957 ISSN: 0036-5521
- DE CAESTECKER JOHN: "Medical therapy for supraesophageal complications of gastroesophageal reflux." AMERICAN JOURNAL OF MEDICINE, vol. 103, no. 5 PART A, 24 novembre 1997 (1997-11-24), pages 138S-143S, XP001159192 ISSN: 0002-9343
- CENGIA GIANPAOLO ET AL: "Nocturnal chronic cough and GERD: Clinical correlations and significance of abnormal proximal esophageal reflux." GASTROENTEROLOGY, vol. 116, no. 4 PART 2, avril 1999 (1999-04), page A134, XP008018958 Digestive Disease Week and the 100th Annual Meeting of the American Gastroenterological Association;Orlando, Florida, USA; May 16-19, 1999 ISSN: 0016-5085
- MAY B ET AL: "Extraesophageal manifestations of reflux disease concerning the airways." VERDAUUNGSKRANKHEITEN, vol. 16, no. 6, novembre 1998 (1998-11), pages 267-270, XP008018331 ISSN: 0174-738X
- WORTH H ET AL: "Interrelationships between gastro-esophageal reflux and airway diseases-pathophysiology, diagnostic and therapeutic possibilities." ATEMWEGS- UND LUNGENKRANKHEITEN, vol. 20, no. 12, 1994, pages 697-700, XP008018329 ISSN: 0341-3055
- RODRIGUEZ-TELLEZ M ET AL: "Posterior laryngitis: Effects of treatment with omeprazole alone." REVISTA ESPANOLA DE ENFERMEDADES DIGESTIVAS, vol. 94, no. 3, 2002, pages 127-130, XP008018960 ISSN: 1130-0108
- WILKINSON S P ET AL: "Regression of columnar-lined (Barrett's) oesophagus with omeprazole 40 mg daily: Results of 5 years of continuous therapy." ALIMENTARY PHARMACOLOGY & THERAPEUTICS, vol. 13, no. 9, 1999, pages 1205-1209, XP002246271 ISSN: 0269-2813
- HENDEL J ET AL: "Morning or evening dosage of omeprazole for gastro-oesophageal reflux disease?" ALIMENTARY PHARMACOLOGY & THERAPEUTICS, vol. 9, no. 6, 1995, pages 693-697, XP008018964 ISSN: 0269-2813
- FITZGERALD R C ET AL: "Review article: Barrett's oesophagus, dysplasia and pharmacologic acid suppression." ALIMENTARY PHARMACOLOGY & THERAPEUTICS MAR 2001, vol. 15, no. 3, mars 2001 (2001-03), pages 269-276, XP002511038 ISSN: 0269-2813
- SCARPIGNATO C: "Acid suppression therapy: where do we go from here?" DIGESTIVE DISEASES, KARGER, BASEL, CH, vol. 24, no. 1-2, 1 janvier 2006 (2006-01-01), pages 11-46, XP008083613 ISSN: 0257-2753
- DOMAGALA FLORENCE ET AL: "Pharmacokinetics of tenatoprazole, a newly synthesized proton pump inhibitor, in healthy male Caucasian volunteers", ARZNEIMITTEL-FORSCHUNG, vol. 56, no. 1, 2006, pages 33-39, XP008142156, ISSN: 0004-4172

## Description

La présente invention, comme définie dans les revendications concerne le traitement de maladies liées au reflux gastro-oesophagien, aux hémorragies digestives et aux dyspepsies, et plus particulièrement l'utilisation du ténatoprazole dans la fabrication d'un médicament destiné au traitement de maladies liées au reflux gastro-oesophagien, aux hémorragies digestives et aux dyspepsies.

Le ténatoprazole, c'est-à-dire la 5-méthoxy-2-[[(4-méthoxy-3,5-diméthyl-2-pyridyl)méthyl]sulfinyl]imidazo[9,5-b]pyridine, est décrit dans le brevet EP 254.588. Il fait partie des médicaments considérés comme des inhibiteurs de la pompe à protons, utiles pour le traitement des ulcères gastriques et duodénaux. Parmi les autres inhibiteurs de la pompe à protons, on peut citer l'oméprazole, le rabéprazole, le pantoprazole, le lansoprazole, qui présentent tous une analogie structurelle, et se rattachent au groupe des pyridinyl-méthyl-sulfinyl-benzimidazoles. Ces composés sont des sulfoxydes présentant une asymétrie au niveau de l'atome de soufre et sont donc généralement sous forme de mélange racémique de deux énantiomères.

Le premier dérivé connu de cette série est l'oméprazole, décrit dans le brevet EP 005.129, qui possède des propriétés inhibitrices de la sécrétion acide gastrique, et est largement utilisé comme anti-ulcéreux en thérapeutique humaine.

L'oméprazole a aussi été envisagé dans le traitement des troubles du reflux gastro-oesophagien, mais son action dans une telle indication n'est pas totalement satisfaisante. Ainsi, des études ont montré que sa durée d'action, comme dans le cas des autres inhibiteurs de la pompe à protons, est insuffisante pour traiter efficacement le reflux nocturne.

Le reflux gastro-oesophagien est considéré comme lié essentiellement à un désordre de la motilité caractérisé par un relâchement transitoire anormalement fréquent et une perte de tonus du sphincter du bas oesophage. Ces anomalies ont pour effet de permettre un reflux du contenu de l'estomac dans l'oesophage. En outre, chez les patients souffrant de reflux gastro-oesophagien, l'élimination de l'acidité du reflux est en moyenne 50% plus lente que chez un sujet normal et la résistance de la paroi oesophagienne à l'agression acide est sensiblement diminuée. Aussi, la sécrétion acide de l'estomac joue un rôle majeur dans l'apparition et la persistance des lésions de la muqueuse oesophagienne des patients souffrant de reflux gastro-oesophagien.

Diverses études ont montré que la sévérité des symptômes chez les patients souffrant de reflux gastro-oesophagien est proportionnelle à la durée d'exposition de la muqueuse oesophagienne à l'acide (Howden CW, Burget DW, Hunt RH "Appropriate acid suppression for optimal healing of duodenal ulcer and gastro-oesophageal reflux disease", Scand. J. Gastroenterol, Suppl (1994) 201:79-82). Ainsi, les sujets non symptomatiques ont une exposition d'environ 1% (pourcentage de temps d'exposition à l'acidité en une journée), tandis que ceux qui sont affectés occasionnellement de reflux gastro-oesophagien ont un taux d'exposition voisin de 2%, les sujets avec symptômes quotidiens un taux de 3% et les patients présentant des lésions endoscopiques un taux variant de 6% à 12% selon la gravité de la lésion. Ces études ont été faites pour des expositions à une acidité de pH inférieur à 4, c'est-à-dire anormalement bas au niveau de l'oesophage où les valeurs normales sont généralement comprises entre 5 et 7.

Ces études ont donc montré que plus l'exposition acide est longue, plus les symptômes et les lésions de la muqueuse oesophagienne sont sévères.

De plus, les études ont montré que la suppression de l'acide résultant d'un traitement médical approprié est corrélée avec le taux de guérison des lésions, les paramètres importants étant la durée de l'inhibition acide et son amplitude. C'est pourquoi on a souvent prescrit aux patients souffrant de reflux gastro-oesophagien, l'administration de médicaments antiacides ou d'antagonistes des récepteurs à l'histamine ou encore d'inhibiteurs de la pompe à protons en vue d'obtenir un soulagement des symptômes. Cependant, la plupart des médicaments utilisés ne sont pas pleinement satisfaisants car ils ne procurent qu'un soulagement partiel des symptômes, ou ils ont une durée d'action trop courte, impliquant des prises répétées de médicament.

De même, dans le traitement de la dyspepsie, les études ont montré que des inhibiteurs de la pompe à protons pouvaient apporter un certain soulagement, mais peu de traitements sont efficaces.

La dyspepsie fonctionnelle est constituée par un ensemble protéiforme de symptômes liés à l'alimentation et associant, à des degrés divers, des douleurs ou une gêne au niveau de la partie haute de l'abdomen, une sensation de satiété précoce ou de digestion lente, des nausées, des vomissements, etc. La physiopathologie de la dyspepsie fonctionnelle est encore aujourd'hui mal connue.

Il a été montré que chez certains patients, surtout ceux souffrant de dyspepsie fonctionnelle pseudo-ulcéreuse ou mimant les symptômes d'un reflux gastro-oesophagien, un soulagement peut être obtenu par l'administration d'un médicament de la classe thérapeutique des inhibiteurs de la pompe à protons, tels que l'oméprazole et le lansoprazole. Le bénéfice thérapeutique, dans ces études, est surtout observé chez les sujets présentant une exposition acide oesophagienne augmentée. Cependant, la demi-vie d'élimination relativement courte des inhibiteurs de la pompe à protons constitue un inconvénient pour la suppression de l'acidité gastrique, et ne permet donc pas de les prescrire efficacement dans le traitement de la dyspepsie fonctionnelle.

Diverses techniques ont été mises au point pour obtenir des formulations contenant des inhibiteurs de la pompe à protons susceptibles de procurer des propriétés améliorées.

Par exemple, WO 02.072070 décrit des microparticules obtenues par une technique de lyophilisation par pulvérisation, possédant une teneur élevée en sel de magnésium d'oméprazole ou d'esoméprazole, qui peuvent être enduites par une couche gastro-résistante pour les protéger du contact avec le jus gastrique acide. WO 99.59544 décrit des comprimés désintégrable pour administration par voie orale contenant de fins granulés comprenant une composition enduite par une couche gastro-résistante. Cette composition comprend une substance active telle que le lansoprazole. Les microparticules et comprimés contenant de fins granulés décrits dans ces documents sont supposés être utiles dans les traitement usuels des affections liées à l'acidité gastrique, mais aucun résultat clinique n'est fourni. Adis R&D Profile (2002:3(4) 276-277) relate certaines propriétés du ténatoprazole et signale qu'il est enregistré au Japon pour le reflux oesophagien en Avril 2002, mais cette information est erronée et l'enregistrement n'a jamais été délivré car la démonstration de cette indication possible du ténatoprazole n'a jamais été faite à l'époque.

Il subsiste donc aujourd'hui un besoin d'un médicament susceptible de traiter et soulager efficacement les patients souffrant de reflux gastro-oesophagien et de dyspepsies.

Les études et expérimentations effectuées par la demanderesse ont montré de manière inattendue que le ténatoprazole pouvait être utilisé efficacement dans le traitement des maladies liées au reflux gastro-oesophagien et aux dyspepsies, alors que l'oméprazole et les autres inhibiteurs de la pompe à protons de structure analogue ne procurent pas une efficacité de traitement satisfaisante dans ces indications.

La présente invention a donc pour objet l'utilisation du ténatoprazole dans la fabrication d'un médicament destiné au traitement séquentiel des symptômes atypiques et oesophagiens du reflux gastro-oesophagien, des hémorragies digestives et des dyspepsies par une seule administration par semaine, par voie orale, d'un seul comprimé dosé à 20 ou 40 mg.

Comme l'oméprazole et les autres sulfoxydes à structure analogue, le ténatoprazole comporte une structure asymétrique et peut donc se présenter sous la forme du mélange racémique ou de ses énantiomères.

Contrairement à tous les autres inhibiteurs de la pompe à protons tels que, par exemple, l'oméprazole ou l'esoméprazole, et de manière inattendue, le ténatoprazole possède une durée d'action nettement prolongée, résultant d'une demi-vie environ 7 fois supérieure. Ainsi, les données médicales recueillies montrent que le ténatoprazole apporte un niveau de soulagement des symptômes et de cicatrisation des lésions supérieur à celui des autres médicaments appartenant à la même classe thérapeutique des inhibiteurs de la pompe à protons, ce qui permet alors son utilisation efficace dans le traitement des symptômes atypiques et oesophagiens du reflux gastro-oesophagien, des hémorragies digestives et des dyspepsies.

La présente invention permet d'utiliser le ténatoprazole pour apporter un niveau supérieur de soulagement des symptômes atypiques du reflux gastro-oesophagien, et plus particulièrement des symptômes atypiques nocturnes qui sont encore aujourd'hui souvent réfractaires aux traitements par les inhibiteurs de la pompe à protons usuels, tels que l'oméprazole. De même, la présente invention apporte un avantage sensible dans le traitement, à la demande, de symptômes atypiques du reflux gastro-oesophagien où le volume de la prise médicamenteuse est conditionné à la durée de l'effet thérapeutique.

Un autre avantage de la présente invention est que le ténatoprazole peut aussi agir efficacement sur la maladie de l'oesophage de Barrett, ou endobrachyoesophage, qui est définie par la présence d'une muqueuse de type intestinal (cylindrique) au niveau du bas oesophage ou de la jonction gastro-oesophagienne. Cette affection est une complication de l'oesophagite peptique, qui peut résulter du reflux gastro-oesophagien, et elle peut dégénérer dans certains cas en adénocarcinome.

Les malades souffrant de l'oesophage de Barrett ont en général un reflux gastro-oesophagien plus important que la moyenne, et l'importance de l'acidité du reflux peut avoir des effets néfastes sur la différentiation et la prolifération cellulaire, pouvant favoriser le développement d'une dysplasie. Il est donc important de pouvoir réduire la sécrétion acide chez les patients présentant des symptômes liés au reflux gastro-oesophagien avec des lésions histologiques en rapport avec un oesophage de Barrett.

Le traitement doit procurer une suppression maximale de l'acidité du reflux gastro-oesophagien, dans le cas de l'oesophage de Barrett, et l'administration de ténatoprazole permet précisément d'y parvenir, et plus particulièrement de prévenir les poussées acides nocturnes, ce que les médicaments actuellement disponibles ne permettent pas d'obtenir, même les inhibiteurs de la pompe à protons classiques.

Comme indiqué ci-dessus, le ténatoprazole se distingue des autres inhibiteurs de la pompe à protons par une demi-vie d'élimination étonnamment plus longue, et aussi par une exposition tissulaire importante, comme l'ont démontré les expérimentations effectuées par la demanderesse.

L'étude de phase I chez des individus de type causasien (n=8 par groupe) a permis de montrer l'influence de différentes doses de ténatoprazole sur les paramètres pharmacocinétiques, dans le cas d'une administration par voie orale en une dose unique, et pendant une période de 7 jours.

Les doses testées sont de 10, 20, 40 et 80 mg de ténatoprazole.

Les résultats obtenus sont regroupés au Tableau 1 ci-après.

**Tableau 1**

| | Dose unique | | | | Dose répétée (7 jours) | | | |
|---|---|---|---|---|---|---|---|---|
| | 10 mg | 20 mg | 40 mg | 80 mg | 10 mg | 20 mg | 40 mg | 80 mg |
| Cmax (µg/ml) | 0,9 | 2,4 | 5,3 | 8,3 | 1,6 | 3 | 5,5 | 11,8 |
| Tmax (h) | 4 | 4 | 3 | 3 | 3 | 2 | 3 | 2 |
| T1/2 (h) | 5 | 6 | 6 | 7 | 5 | 8 | 9 | 9,2 |
| AUC 0-t | 8 | 24 | 43 | 97 | 13 | 36 | 75 | 218 |

Dans ce tableau, les abréviations utilisées ont les signification suivantes :
Cmax concentration maximale
Tmax temps pour obtenir la concentration maximale
T1/2 temps de demi-vie d'élimination
AUC₀₋ₜ aire sous la courbe, entre le temps 0 et la dernière concentration mesurable.

Les résultats exposés au Tableau 1 ci-dessus montrent que les moyennes de temps de demi-vie d'élimination sont comprises entre 5 et 6 heures après administration d'une dose unique, et entre 5 et 9,5 heures après 7 jours d'administration, selon la dose. Le ténatoprazole présente aussi de fortes valeurs d'AUC (aire sous la courbe) mettant en évidence un faible taux de métabolisme et/ou une forte biodisponibilité par voie orale. De plus, quelles que soient les conditions d'administration, unique ou répétée, les valeurs de Cmax, AUC₀₋ₜ et AUC_{0-inf} augmentent de manière linéaire. La valeur de AUC_{0-inf} est calculée par extrapolation.

Une comparaison des valeurs d'AUC entre deux inhibiteurs de la pompe à protons, le lansoprazole et l'oméprazole, a déjà été faite par Tolman et al. (J. Clin. Gastroenterol., 24(2), 65-70, 1997) mais elle ne permet pas de juger de la supériorité d'un produit par rapport à un autre. En effet, différents critères entrent en jeu, à savoir le temps de régénération de la pompe, et le temps passé au-dessus de la concentration minimale nécessaire pour inhiber les pompes à protons. En ce qui concerne le temps de régénération des pompes, on observe que les pompes ont généralement une durée de demi-vie de l'ordre de 30 à 48 heures, et elles sont donc renouvelées totalement toutes les 72 à 96 heures.

Grâce aux propriétés pharmacocinétiques exposées ci-dessus, le ténatoprazole permet de s'opposer au phénomène de régénération des pompes à protons en maintenant une concentration inhibitrice sur une période de temps suffisamment longue pour répondre aux deux critères précités.

Ainsi, l'exposition prolongée liée à la longue demi-vie d'élimination du ténatoprazole, mise en évidence par la valeur d'AUC, lui confère une plus longue présence au niveau des sites d'action et procure donc un effet pharmacodynamique prolongé dans le temps. Les expérimentations montrent ainsi que le ténatoprazole possède un rapport demi-vie plasmatique / temps de régénération des pompes notablement plus élevé que celui des autres inhibiteurs de la pompe à protons, ce qui permet de l'utiliser dans des pathologies où les médicaments actuels sont peu efficaces, en particulier dans le traitement des symptômes atypiques et oesophagiens du reflux gastro-oesophagien, des dyspepsies et de l'oesophage de Barrett.

Plus particulièrement, suivant la présente invention, le ténatoprazole peut être utilisé pour le traitement de symptômes atypiques du reflux gastro-oesophagien tels que l'asthme et les accès dyspnéiques de type asthmatiforme, la pharyngite, la dysphonie, le pseudo-angor, la toux paroxystique et la toux nocturne. Il est aussi particulièrement efficace dans le traitement de la dyspepsie pseudo-ulcéreuse. Comme indiqué plus haut, il peut également être utilisé avec succès dans le traitement de l'oesophage de Barrett.

Le ténatoprazole, dans le traitement des symptômes atypiques et oesophagiens du reflux gastro-oesophagien, des hémorragies digestives, notamment d'origine ulcéreuse, et des dyspepsies, peut être administré sous les formes usuelles adaptées au mode d'administration choisi, par exemple par voie orale ou parentérale, de préférence par voie orale ou intraveineuse. On peut utiliser par exemple des formulations de comprimés ou de gélules contenant le ténatoprazole comme principe actif, ou encore des solutés buvables ou des émulsions ou solutions pour administration parentérale contenant un sel de ténatoprazole avec un support pharmaceutiquement acceptable usuel. Le sel de ténatoprazole peut être choisi parmi les sels de sodium, de potassium, de magnésium ou de calcium.

A titre d'exemple, une formulation appropriée de comprimés contenant 20 mg de ténatoprazole associé à des supports et excipients pharmaceutiquement acceptables, est indiquée ci-dessous :

| | | |
|---|---|---|
| Ténatoprazole | | 20,0 mg |
| lactose | | 32,0 mg |
| hydroxyde d'aluminium | | 17,5 mg |
| hydroxypropylcellulose | | 12,1 mg |
| talc | | 4,5 mg |
| dioxyde de titane | | 3,2 mg |
| stéarate de magnésium | | 1,0 mg |
| excipients usuels | q.s.p. | 160 mg |

La posologie est déterminée par le praticien en fonction de l'état du patient et de la gravité de l'affection. L'invention présente l'avantage de permettre un traitement séquentiel efficace par simple administration d'un seul comprimé dosé à 20 ou 40 mg par semaine.

Des exemples cliniques sont donnés ci-après, montrant les effets du traitement sur des patients souffrant de reflux gastro-oesophagien ou de dyspepsie, traités par administration de ténatoprazole par voie orale. Il est à noter que la posologie utilisée dans les exemples est différente de celle des revendications.

**Tableau 2**

| Traitement de patients avec symptômes de reflux gastro-oesophagien | | | | |
|---|---|---|---|---|
| Age/Sexe | Symptôme prédominant | Durée de traitement | Evolution du symptôme | Tolérance |
| 47/F | b.n. | 8 semaines | ++ | +++ |
| 38/M | b. | 8 semaines | +++ | +++ |
| 35/F | b.n. | 4 semaines | ++ | +++ |
| 34/M | b. | 8 semaines | +++ | +++ |
| 45/M | b.n. | 8 semaines | +++ | +++ |
| 30/M | b.n. | 8 semaines | +++ | ++ |
| 49/F | r. | 8 semaines | ++ | +++ |
| 42/M | b. | 8 semaines | ++ | +++ |
| 38/F | b.n. | 8 semaines | +++ | +++ |
| 25/F | b. | 12 semaines | +++ | +++ |
| 28/M | b.n. | 4 semaines | +++ | +++ |
| 39/F | b.n. | 4 semaines | + | +++ |
| 41/M | b. | 8 semaines | +++ | +++ |
| 36/F | r. | 8 semaines | +++ | ++ |

| | | | | |
|---|---|---|---|---|
| b. : brûlures b.n.: brûlures nocturnes r. régurgitations | | | | |

Les symboles + à +++ identifient une évolution du symptôme et une tolérance de valeur moyenne à très favorable.

Le traitement consiste en une administration quotidienne d'un comprimé dosé à 20 mg de ténatoprazole. Le tableau montre que le traitement est parfaitement toléré dans 12 cas sur 14 et bien toléré dans les deux autres, tandis que l'évolution constatée des symptômes a été généralement très favorable.

**Tableau 3**

| Traitement de patients avec symptômes atypiques de reflux gastro-oesophagien | | | | | |
|---|---|---|---|---|---|
| Age/Sexe | Symptôme prédominant | liaison avec gerd | Durée de traitement | Evolution du symptôme | Tolérance |
| 44/M | pharyngite | + | 4 semaines | ++ | +++ |
| 36/M | dysphonie | ++ | 5 semaines | +++ | +++ |
| 34/F | dysphonie | ++ | 4 semaines | ++ | +++ |
| 45/M | pseudo angor | ++ | 8 semaines | +++ | +++ |
| 29/F | toux nocturne | +++ | 7 semaines | +++ | +++ |
| 27/M | carie dentaire | + | 12 semaines | 0 | ++ |
| 33/M | asthme | ++ | 12 semaines | ++ | +++ |
| 34/F | pharyngite | ++ | 8 semaines | ++ | +++ |
| 36/F | toux nocturne | ++ | 8 semaines | +++ | ++ |
| 26/M | asthme | ++ | 12 semaines | +++ | +++ |
| 49/M | pseudo angor | ++ | 12 semaines | +++ | +++ |
| 31/F | pharyngite | + | 8 semaines | + | +++ |

| | | | | | |
|---|---|---|---|---|---|
| gerd : troubles du reflux gastro-oesophagien. | | | | | |

Les résultats du tableau ci-dessus montrent que l'évolution des symptômes est particulièrement favorable dans les cas où le lien avec le reflux gastro-oesophagien est plus net.

**Tableau 4**

| Traitement de patients avec symptômes de dyspepsie fonctionnelle | | | | |
|---|---|---|---|---|
| Age/Sexe | Symptôme prédominant | Durée de traitement | Evolution du symptôme | Tolérance |
| 47/F | n. | 4 semaines | ++ | +++ |
| 38/M | p.g. | 8 semaines | +++ | +++ |
| 35/F | b. | 8 semaines | +++ | +++ |
| 34/F | s.p. | 8 semaines | +++ | +++ |
| 45/M | d.e. | 6 semaines | +++ | ++ |
| 30/F | b.n. | 8 semaines | +++ | +++ |
| 49/F | n. | 8 semaines | ++ | +++ |
| 42/M | s.p. | 6 semaines | ++ | +++ |
| 38/F | d.e. | 8 semaines | +++ | ++ |
| 25/F | g.e. | 12 semaines | ++ | +++ |
| 28/M | s.b. | 4 semaines | + | +++ |
| 39/F | d.e. | 4 semaines | ++ | ++ |
| 41/M | b. | 6 semaines | +++ | +++ |
| 36/F | g.e. | 8 semaines | +++ | ++ |
| 44/F | n. | 10 semaines | +++ | +++ |

| | | | | |
|---|---|---|---|---|
| b. : brûlures b.n.: brûlures nocturnes n. : nausées p.g.: sensations de plénitude gastrique s.p.: satiété précoce d.e.: douleur épigastrique g.e.: gêne épigastrique s.b.: sensation de ballonnement | | | | |

Ces résultats confirment l'efficacité du ténatoprazole, administré conformément à l'invention, dans le traitement de la dyspepsie.

Deux études ouvertes ont été réalisées pour évaluer l'efficacité et la sécurité du ténatoprazole dans le traitement du reflux gastro-oesophagien. A 22 patients dans la première étude et 24 patients dans la seconde, âgés de plus de 20 ans, souffrant de reflux oesophagien érosif et/ou ulcéré (diagnostiqué par endoscopie), on a administré des comprimés de granulés à enrobage gastro-résistant contenant 10 mg de ténatoprazole.

Les comprimés ont été administrés par voie orale, une fois par jour après le petit déjeuner, pendant une période de traitement de 8 semaines, qui a été prolongée jusqu'à 12 semaines dans certains cas. La guérison a été suivie par examen endoscopique, 4 semaines et 8 semaines après la première administration, ou au retrait du cas de l'étude, les degrés de la maladie ont été évalués suivant la classification de Savary et Miller, et le traitement a été arrêté quand la guérison était confirmée. L'état a été évalué comme guéri quand la disparition de l'érosion était confirmée.

Le degré d'amélioration endoscopique a été évalué suivant les 6 niveaux suivants du degré de l'affection : "guéri", "fortement diminué", modérément diminué", "légèrement diminué", "inchangé" et "aggravé".

Le degré d'amélioration des symptômes subjectifs et objectifs, comparés avec ceux observés au départ de l'étude, a été évalué suivant les 6 niveaux suivants : "fortement amélioré", "modérément amélioré", "légèrement amélioré", "inchangé", "aggravé" et "aucun symptôme depuis le début de l'étude".

La guérison a été observée à 4 semaines et l'administration du ténatoprazole a été arrêtée à ce moment dans 20 cas dans la première étude et 23 cas dan la seconde. Un seul patient n'a pas été guéri au bout de 8 semaines de traitement.

Les résultats sont indiqués aux Tableaux 5 et 6 ci-après.

**Tableau 5**

| **Amélioration du Grade Endoscopique** | | | | | | | |
|---|---|---|---|---|---|---|---|
| | A | B | C | D | E | F | Total |
| 1^{ère} étude | | | | | | | |
| 4 sem. | 20 | 1 | 0 | 0 | 1 | 0 | 22 |
| 8 sem. | 21 | 0 | 0 | 0 | 1 | 0 | 22 |
| terme | 21 | 0 | 0 | 0 | 1 | 0 | 22 |

| 2^{éme} étude | | | | | | | |
|---|---|---|---|---|---|---|---|
| 4 sem. | 23 | 2 | 0 | 0 | 0 | 0 | 25 |
| 8 sem. | 24 | 0 | 0 | 0 | 0 | 0 | 24 |
| 12 sem. | 24 | 0 | 0 | 0 | 0 | 0 | 24 |
| terme | 24 | 0 | 0 | 0 | 0 | 0 | 24 |

| | | | | | | | |
|---|---|---|---|---|---|---|---|
| A : guéri B : fortement diminué C : modérément diminué D : légèrement diminué E : inchangé F : aggravé | | | | | | | |

**Tableau 6**

| **Amélioration du niveau d'intensité des symptômes** | | | | | | |
|---|---|---|---|---|---|---|
| | A | B | C | D | E | Total |
| 1^{ère} étude | | | | | | |
| 1 sem. | 16 | 6 | 0 | 3 | 0 | 25 |
| 2 sem. | 21 | 1 | 0 | 1 | 0 | 23 |
| 4 sem. | 23 | 0 | 1 | 0 | 0 | 24 |
| 6 sem. | 10 | 0 | 1 | 0 | 0 | 11 |
| 8 sem. | 9 | 0 | 1 | 0 | 0 | 10 |
| 10 sem. | 2 | 0 | 0 | 0 | 0 | 2 |
| Final | 21 | 0 | 1 | 0 | 0 | 22 |

| 2^{ème} study | | | | | | |
|---|---|---|---|---|---|---|
| 1 sem. | 19 | 2 | 1 | 1 | 0 | 23 |
| 2 sem. | 21 | 1 | 1 | 0 | 0 | 23 |
| 4 sem. | 20 | 1 | 1 | 0 | 0 | 22 |
| 6 sem. | 10 | 0 | 1 | 0 | 0 | 11 |
| 8 sem. | 10 | 1 | 0 | 0 | 0 | 11 |
| 10 sem. | 0 | 1 | 0 | 0 | 0 | 1 |
| 12 sem. | 0 | 1 | 0 | 0 | 0 | 1 |
| Final | 19 | 2 | 0 | 0 | 0 | 21 |

| | | | | | | |
|---|---|---|---|---|---|---|
| A : fortement amélioré B : modérément amélioré C : légèrement amélioré D : inchangé E : aggravé | | | | | | |

Les résultats ci-dessus démontrent que le ténatoprazole est très efficace dans le traitement du reflux gastro-oesophagien, car la guérison est obtenue par un traitement de 4 semaines, à comparer aux résultats insatisfaisants obtenus dans des traitements de 8 semaines avec les inhibiteurs de la pompe à protons usuels.

## Revendications

1. Utilisation du ténatoprazole dans la fabrication d'un médicament pour le traitement séquentiel des symptômes atypiques et oesophagiens du reflux gastro-oesophagien, des hémorragies digestives et des dyspepsies par une seule administration par semaine, par voie orale, d'un seul comprimé dosé à 20 ou 40 mg.

2. Utilisation selon la revendication 1, **caractérisée en ce que** le ténatoprazole est présenté en dose unitaire contenant 20 ou 40 mg de principe actif, associé à un ou plusieurs excipients et supports pharmaceutiquement acceptables.

3. Utilisation selon l'une quelconque des revendications 1 et 2, **caractérisée en ce que** le médicament est destiné au traitement de l'oesophage de Barrett.

4. Utilisation selon l'une quelconque des revendications 1 et 2, **caractérisée en ce que** le médicament est destiné au traitement du reflux nocturne.

5. Utilisation selon l'une quelconque des revendications 1 et 2, **caractérisée en ce que** le médicament est destiné au traitement de la dyspepsie pseudo-ulcéreuse.

6. Utilisation selon l'une quelconque des revendications 1 et 2, **caractérisée en ce que** le médicament est destiné au traitement de l'asthme et des accès dyspnéiques de type asthmatiforme, la pharyngite, la dysphonie, le pseudo-angor, la toux paroxystique et la toux nocturne.

7. Utilisation selon l'une quelconque des revendications précédentes, **caractérisée en ce que** le ténatoprazole est sous forme de sel de sodium, de potassium, de magnésium ou de calcium.

## Claims

1. The use of tenatoprazole in the manufacture of a medicament for the treatment of atypical and oesophageal symptoms of gastroesophageal reflux, digestive bleeding and dyspepsia by a single administration per week, by oral route, of a single 20 or 40 mg dosed tablet.

2. Use according to claim 1, wherein tenatoprazole is presented in a unit dosage form containing 20 to 40 mg of active substance, associated with one or several pharmaceutically acceptable excipients and substrates.

3. Use according to any of claims 1 and 2, wherein the medicament is intended for the treatment of Barrett's oesophagus.

4. Use according to any of claims 1 and 2, wherein the medicament is intended for the treatment of nocturnal reflux.

5. Use according to any of claims 1 and 2, wherein the medicament is intended for the treatment of pseudo-ulcer dyspepsia.

6. Use according to any of claims 1 and 2, wherein the medicament is intended for the treatment of asthma, asthma-like acute dyspnoea, pharyngitis, dysphonia, pseudo-angina, paroxysmal cough and nocturnal cough.

7. Use according to any of the preceding claims, wherein the medicament is presented as a salt of sodium, potassium, magnesium or calcium.

## Patentansprüche

1. Verwendung von Tenatoprazol bei der Herstellung eines Arzneimittels zur sequentiellen Behandlung von atypischen und ösophagealen Symptomen des gastroösophagealen Refluxes, von Blutungen des Verdauungssystems und Verdauungsstörungen durch eine einzige Verabreichung pro Woche auf oralem Weg einer einzigen auf 20 oder 40 mg dosierten Tablette.

2. Verwendung nach Anspruch 1, **dadurch gekennzeichnet, dass** das Tenatoprazol in Einheitsdosis vorhanden ist, die 20 oder 40 mg Wirkprinzip beinhaltet, verbunden mit einem oder mehreren pharmazeutisch akzeptablen Hilfs- und Trägerstoffen.

3. Verwendung nach einem der Ansprüche 1 und 2, **dadurch gekennzeichnet, dass** das Arzneimittel zur Behandlung der Barrett-Speiseröhre bestimmt ist.

4. Verwendung nach einem der Ansprüche 1 und 2, **dadurch gekennzeichnet, dass** das Arzneimittel zur Behandlung des nächtlichen Refluxes bestimmt ist.

5. Verwendung nach einem der Ansprüche 1 und 2, **dadurch gekennzeichnet, dass** das Arzneimittel zur Behandlung der pseudoulzerösen Verdauungsstörung bestimmt ist.

6. Verwendung nach einem der Ansprüche 1 und 2, **dadurch gekennzeichnet, dass** das Arzneimittel zur Behandlung des Asthmas und der asthmatischen Kurzatmigkeitsanfälle, der Pharyngitis, der Dysphonie, der Pseudo-Beklemmung, des paroxystischen Hustens und des nächtlichen Hustens bestimmt ist.

7. Verwendung nach einem der vorangehenden Ansprüche, **dadurch gekennzeichnet, dass** das Tenatoprazol in Form von Natrium-, Kalium-, Magnesium- oder Kalziumsalz ist.
